(19) Europäisches Patentamt
European Patent Office
Office européen des brevets

(11) Publication number: **0 054 283**
**A1**

(12) # EUROPEAN PATENT APPLICATION

(21) Application number: 81110377.9

(22) Date of filing: 11.12.81

(51) Int. Cl.³: C 07 D 323/00
C 07 D 498/08, C 07 D 273/00
C 07 D 327/00

(30) Priority: 12.12.80 US 215823

(43) Date of publication of application: 23.06.82 Bulletin 82/25

(84) Designated Contracting States: AT BE CH DE FR GB IT LI NL SE

(71) Applicant: UNION CARBIDE CORPORATION
Law Department - Patent Section Old Ridgebury Road
Danbury, CT 06817(US)

(72) Inventor: Harrison, Arnold Myron
1621 Janet Place
South Charleston West Virginia 25303(US)

(72) Inventor: Kaplan, Leonard
227 Walker Drive
Dunbar West Virginia 25064(US)

(74) Representative: Schmied-Kowarzik, Volker, Dr. et al,
Siegfriedstrasse 8
D-8000 München 40(DE)

(54) **Process for preparing substituted macrocyclics.**

(57) Substituted macrocyclics are prepared from a macrocyclic substrate having a carbon-hydrogen bond adjacent to a hetero ring atom and a substituent-forming compound in the presence of a chemical initiator.

EP 0 054 283 A1

# PROCESS FOR PREPARING SUBSTITUTED MACROCYCLICS

This invention relates to a process for the preparation of substituted macrocyclic compounds using substituent-forming compounds and a chemical initiator.

The use of macrocyclic compounds, e.g., crown ethers and cryptands, as complexing agents is well known. (See "Synthetic Multidentate Macrocyclic Compounds", edited by Reed M. Izatt and James J. Christensen, Academic Press, New York, 1978; and Progress in Macrocyclic Chemistry, Volume 1," edited by Reed M. Izatt and James J. Christensen, John Wiley and Sons, New York, 1979).

The interest in the novel chemical nature and utility of these macrocyclic compounds has led to increased interest in the chemistry and synthesis of substituted macrocyclics. In fact, several reports in the prior art suggest that substituted macrocyclic compounds may be very valuable. (See "Synthetic Multidentate Macrocyclic Compounds", edited by Reed M. Izatt and James J. Christensen, Academic Press, New York, 1978; "Progress in Macrocyclic Chemistry, Volume 1," edited by Reed M. Izatt and James J. Christensen, John Wiley and Sons, New York, 1979, J. S. Bradshaw and P. E. Stott, Tetrahedron, 36, 461 (1980)).

The free radical additions of both aliphatic and cyclic ethers to a variety of unsaturated substrates have been reported. See M. D. Peterson and A. G. Weber, "Ethylene-Acetal Reaction Product," U. S. Patent 2,395,292 (1946); "Terminally Substituted Polymerization Products of Ethylene," Brit. Patent 583,181 (1946) [Chem. Abstr. 41, 2937 g (1947)]; W. E. Hanford and J. R. Roland, "High Molecular Weight Products of Ethylene," U. S. Patent 2,402,137 (1946); W. E. Hanford, "Organic Fluoroethers and Their Preparation," U. S. Patent 2,433,844 (1948); T. M. Patrick, Jr., "(Dimethoxymethyl)succinic Anhydride and Telomeric Products," U. S. Patent 2,628,238 (1953)[Chem. Abstr., 48, 711e (1954)]; T. M. Patrick, Jr., "Cyclic Acetal Telomers," U. S. Patent 2,684,373 (1954) [Chem. Abstr., 49, 7602i (1955)], T. M. Patrick, Jr., "Acetal Telomers," U. S. Patent 2,716,660 (1955) [Chem. Abstr., 49, 16523h (1955)]; R. I. Longley, Jr., and T. M. Patrick, Jr., "Process for Producing α-Alkoxybenzylsuccinic Anhydrides and Nuclearly Substituted Benzyl Derivatives Thereof," U. S. Patent 2,841,592 (1958); C. H. McDonnell, "Free Radical Reactions of Ethers," Ph.D. Thesis, University of Chicago, 1955; C. F. Feasley, W. E. Garwood, A. N. Sachanen, and F. M. Seger, "Synthetic Lubricants," U. S. Patent 2,743,281 (1956) [Chem. Abstr., 50, 11012e (1956)]; G. O. Schenck and H. Formanek, "On the Radiation Chemistry of Azodicarboxylic Esters," Angew. Chem., 70, 505 (1958); I. I. Shuikin and B. L. Lebedev, "Alkylation of Tetrahydrofuran by Ethylene," Dokl.Akad. Nauk SSSR,

139, 131(1961); N. I. Shiukin and B. L. Lebedev, "Thermal Alkylation of Tetrahydro-2-methylfuran with Ethylene," Izv. Akad. Nauk SSSR, 2195(1961), N. I. Shiukin and B. L. Lebedev, "Alkylation of Tetrahydrofuran by Ethylene in the Presence of Di-tert-butyl Peroxide," Izv. Akad. Nauk SSSR, 533(1962); N. I. Shuikin and B. L. Lebedev, "On the Alkylation of Cyclic Ethers," Z. Chem., 6, 459 (1966), N. I. Shuikin and B. L. Lebedev, "Alkylation of Tetrahydrofuran with Alkenes and Unsaturated Ethers," Izv. Akad. Nauk SSSR, 639(1967); N. I. Shuikin, B. L. Lebedev, and I. P. Yakovlev, "Alkylation of Tetrahydropyran, "Izv. Akad. Nauk SSSR, 644(1967); T. J. Wallace and R. J. Gritter, "The Free Radical Chemistry of Cyclic Ethers, III. A Free Radical Rearrangement Reaction," J. Org. Chem., 27, 3067(1962); T. J. Wallace, R. J. Gritter, and H. G. Walsh, "Free Radical Chemistry of Cyclic Ethers: p-Dioxane and Morpholine," Nature, 198, 284(1963); J.Diekmann and C. J. Pedersen, "Photaddition of Tetrahydrofuran to 7,7,8,8-Tetracyanoquinodimethane and Tetracyanoethylene," J. Org. Chem., 28, 2879(1963); R. L. Jacobs and G. G. Ecke, "Free Radical Addition of Cyclic Ethers to Maleic Anhydride," ibid., p. 3036; T. Matsuda, K. Yumoto, and K. Iseda, Abstr., Symp. on Isotopes of the 4th Meeting, Tokyo, 1963 [cited in H. Muramatsu, K. Inukai, and T. Ueda, J. Org. Chem., 29, 2220(1964). A study of the radiation-induced addition of cyclic ethers to tetrachloroethylene], T. Matsuda

and T. Yumoto, "Chlorine-Containing Cyclic Ethers," Japan Patent 4708(1966) [Chem. Abstr., 65, 726b(1966)]; T. Yumoto and T. Matsuda, "Synthesis of Oxygen-Containing Halogenated Compounds. III. γ-Ray Induced Addition Reaction of Cyclic Ethers to Tetrachloroethylene," Nippon Kagaku Kaishi, 556(1973) [Chem. Abstr., 79, 4592y(1973)]; T. Yumoto, "Synthesis of Oxygen-Containing Halogenated Compounds. IV. Radical Induced Substitution Reaction between Aliphatic Ethers and Tetrachloroethylene," ibid., p. 1724 [Chem. Abstr., 79, 125740d (1973)]; D. Elad and R. D. Youssefzeh, "The Photoaddition of Cyclic Ethers to 1-Octene," J. Org. Chem., 29, 2031(1964); I. Rosenthal and D. Elad, "The Photoalkylation of Cyclic Ethers," Tetrahedron, 23, 3193(1967); H. Muramatsu, K. Inukai, and T. Ueda, "The Radiation-Induced Addition Reaction of Ethers to Chlorofluoroolefins," J. Org. Chem., 29, 2220(1964); H. Muramatsu, S. Moriguchi, and K. Inukai. "The Addition Reactions of Aldehydes, Alcohols, and Ethers to Perfluorocyclobutene," J. Org. Chem., 31, 1306(1966); H. Muramatsu and K. Inukai, "The Radiation-Induced Addition Reaction of Ethers to 1,2-Dichlorotetrafluorocyclobutene and 1,2-Dichlorohexafluorocyclopentene." ibid., 30, 544(1965); H. Muramatsu, K. Inukai, and T. Ueda, "The Radiation-induced Addition Reactions of Ethers and Alcohols to Perfluoropropene," Bull. Chem. Soc. Japan, 40, 903(1967); R. Askani, "On the Reaction of 1,3-Cyclohexadiene with Diethyl Azodicarboxylate," Chem. Ber., 98, 2551(1965); R. C. Cookson, I. D. R. Stevens,

and C. T. Watts, "Photochemical Reaction of Diethyl Azodicarboxylate with Ethers and Alcohols," Chem. Comm., 259(1965); R. Srinivasan and K. H. Carlough, "Light-induced Addition of Acetylene to Saturated Compounds," Can. J. Chem., 45, 3209(1967); V. Dědek and J. Fikar, "Chemistry of Organic Fluorine Compounds. V. Addition of Diethyl Ether and Tetrahydrofuran to Trifluorochloroethylene," Coll. Czech. Chem. Communs., 34, 3769(1969); P. Singh, "Photoaddition of Dimethyl Acetylenedicarboxylate to Cyclic Ethers," Tet. Lett., 2155(1970); P. Singh, "Photoaddition Reactions. II. Photoaddition of Dimethyl Acetylenedicarboxylate to Cyclic Ethers," J. Org. Chem., 37, 836(1972); H. Hasegawa, M. Tokuriki, and T. Satake, "Photoreactions of Acetylenecarboxylic Esters with Ethers," Nippon Kagaku Kaishi, 748(1972) [Chem. Abstr., 77, 18926f(1972)]; G. Ahlgren, "Reactions of Lone Pair Electron Donors with Unsaturated Electrophiles. I. The Addition of Tetrahydrofuran and Oxetane to Dimethyl Acetylenedicarboxylate," J. Org. Chem., 38, 1369(1973); E. Montaudon and R. Lalande, "Radical Additions. XII - Addition of Oxygenated Heterocycles to Free Acetylenes," Bull. Soc. Chim. France, 2635(1974); E. Montaudon, J. Thépénier, and R. Lalande, "Radical Additions of Oxygenated Heterocycles to 1,2-Hexadiene," Compt. rend., 280C, 1223(1975); E. Montaudon, J. Thépénier, and R. Lalande, "Determination

of the Structure of a Dehydrodimer Produced by Free Radical Addition of Tetrahydrofuran to Propadiene," Compt. rend., 284C, 581(1977); E. Montaudon, J. Thépénier, and R. Lalande, "Radical Additions - XV. Addition of Tetrahydrofuran to Propadiene," Tetrahedron, 34, 897(1978); E. Montaudon, J. Thépénier, and R. Lalande, "Radical Additions. 16. Additions of Oxygenated Heterocycles to Allenes," J. Heterocyclic Chem., 16, 105(1979); T. N. Abroskina, A. D. Sorokin, R. V. Kudryavtsev, and Yu. A. Cheburkov, "Alkylation of Tetrahydrofuran with Fluoroolefins," Izv. Akad. Nauk SSSR, 1741(1974); L. Schmerling, "Free Radical-Induced Monoethylation with Ethylene," A.C.S. Symp. Ser. No. 55, 147(1977); D. L. Rakhmankulov, S. S. Zlotskii, N. E. Maksimova, V. N. Uzikova, S. N. Zlotskii, E.Kh. Kravets, and O. G. Safiev, USSR Patent 568,649(1977)[Chem. Abstr., 87, 152987m(1977)]; Yu. N. Ogibin, M. N. Élinson, G. I. Nikishin, V. I. Kadentsev, and O. S. Chizhov, "Homolytic Reactions of Dibutyl Ether with Dialkylmaleates," Izv. Akad. Nauk SSSR, 1850(1978).

That the methods of the aforementioned reports on the alkylation of ethers were not considered suitable for the alkylation of macrocyclic compounds is evident from the efforts expended in search of a synthetic route to these valuable products. The synthesis of long chain alkyl substituted crown ethers has been reviewed [C. J. Pedersen, in "Synthetic Multidentate Macrocyclic Compounds," ed. R. M. Izatt and J. J. Christensen, Academic Press, 1978, p. 33]:

"There are three general methods of preparing substituted crown compounds:

(1) using intermediates carrying the substituents;
(2) running substitution reactions on the polyethers; and
(3) converting the substituents on the polyethers into others.

In the first case, the substituents must withstand the reaction conditions for the synthesis of the crown compounds and not interfere with the reaction. Hydroxyl and hydrocarbon groups are permissible on aliphatic intermediates and unreactive nonfunctional groups on the aromatic rings. Functional groups, such as carboxyl and extra phenolic hydroxyl groups, must not be present unless they can be protected.

The second method is more applicable to aromatic polyethers than the others because the former will undergo substitution reactions characteristic for aromatic ethers, such as veratrole, provided the conditions do not lead to the scission of the ether linkage (Pedersen, 1967b). They have been halogenated (Shchori and Jagur-Grodzinski, 1972b), nitrated, (Feigenbaum and Michel, 1971), and sulfonated (Unpublished results).

By the third method, carboxyl group has been obtained from methyl, and amino from nitro (Feigenbaum and Michel, 1971). If the substituents do not poison the catalyst and are not removed, substituted aromatic crown compounds can be transformed into the corresponding saturated compounds by catalytic hydrogenation. Some substituents will be reduced simultaneously, such as nitro to amino group."

Note that the second method is not stated to apply to the synthesis of long-chain alkyl-substituted crown ethers. Such synthesis has generally been carried out by employing a multistep synthesis. For the use and synthesis of such compounds, see J. Cooper and P. H. Plesch, "A New Method of Making New Crown Ethers," Chem. Comm., 1017(1974); M. Cinquini, F.

Montanari, and P. Tundo, "Alkyl Substituted Aza-Macrobicyclic Polyethers: Highly Efficient Catalysts in Two-phase Reactions," Chem. Comm., 393(1975); M. Cinquini, S. Colonna, H. Molinari, F. Montanari, and P. Tundo, "Heterogeneous Phase-Transfer Catalysts: Onium Salts, Crown Ethers, and Cryptands Immobilized on Polymer Supports, ibid., 394(1976); M. Cinquini and P. Tundo, Synthesis, 516(1976); M. Cinquini, F. Montanari, and P. Tundo, "Phase-Transfer Catalysts: Synthesis and Catalytic Activity of Alkyl Substituted Azamacrobicyclic Polyethers," Gazz. Chim. Ital., 107, 11(1977); H. Molinari, F. Montanari, and P. Tundo, "Heterogeneous Phase-Transfer Catalysts: High Efficiency of Catalysts Bonded by a Long Chain to a Polymer Matrix," Chem. Comm., 639(1977); D. Landini, A. Maia, F. Montanari, and P. Tundo, "Lipophilic[2.2.2] Cryptands as Phase-Transfer Catalysts. Activation and Nucleophilicity of Anions in Aqueous-Organic Two-Phase Systems and in Organic Solvents of Low Polarity," J. Am. Chem. Soc., 101, 2526(1979); F. Montanari and P. Tundo, "Hydroxymethyl 18-Crown-6 and Hydroxymethyl[2.2.2] Cryptand: Versatile Derivatives for Binding the Two Polyethers to Lipophilic Chains and to Polymer Matrices," Tet. Lett., 5055(1979); R. Humphrey-Baker, M. Grätzel, P. Tundo, and E. Pelizzetti, "Complexes of Nitrogen-Containing Crown Ether Surfactants with Stable Silver Atoms," Angew. Chem. Int. Edit., 18, 630(1979);

K. Monserrat, M. Grätzel, and P. Tundo, "Light-Induced Charge Injection in Functional Crown Ether Vesicles," J. Am. Chem. Soc., 102, 5527(1980); Y. Moroi, E. Pramauro, M. Grätzel, E. Pelizzetti, and P. Tundo, "Surface Activity and Micelle Formation of Alkyl-Substituted Aza-Crown-Ethers and Their Metal Ion Complexes," J. Colloid Interface Sci., 69, 341(1979); D. Clement, F. Damm, and J.-M. Lehn, "Lipophilic Cryptates; Salt Solubilization and Anion Activation," Heterocycles, 5, 477(1976); M. Okahara, M. Miki, S. Yanagida, I. Ikeda, and K. Matsushima, "Synthesis of Polyethylene Glycol β-Haloalkyl Ethers. A New Synthetic Route to Alkyl-Substituted Crown Ethers," Synthesis, 854(1977); M. Tomoi, O. Abe, M. Ikeda, K. Kihara, and H. Kakiuchi, "Syntheses of Hydroxy Group-Containing Crown Ethers and Polymer-Supported Crown Ethers," Tet. Lett., 3031(1978); M. Okahara, I. Ikeda, and S. Yanagida, "Production Method for Crown Ethers," Japanese Patent Publication 98985(1978); T. Mizuno, Y. Nakatsuji, S. Yanagida, and M. Okahara, "The Synthesis and Cation-Complexing Ability of Alkyl Crown Ethers," Bull. Chem. Soc. Japan, 53, 481(1980); M. Okahara, P.-L. Kuo, S. Yamamura, and I. Ikeda, "Effect of Metal Salts on the Cloud Point of Alkyl Crown Compounds," Chem. Comm., 586(1980); W. H. Rastetter and D. P. Phillion, "A Crown Ether, Template-Driven, Macrolide Closure," Tet. Lett., 1469(1979); W. H. Rastetter and D. P. Phillion, "Transition-State-Stabilized Macrolide Closure," J. Org. Chem., 45

1535(1980); T. Kuwamura and T. Kawachi, "Surface Active Crown Ethers. Part 1. Macrocyclic Polyethers of the Acetal Type with Long Alkyl Chains," Yukagaku, 28, 195(1979); J. Le Moigne and J. Simon, "A New Type of Surfactant. The Annelides. Characterization of Organized Metal Ion Assemblies Obtained by Cationic Complexation at the Micelle Subsurface," J. Phys. Chem., 84, 170(1980); P. E. Stott, J. S. Bradshaw, and W. W. Parish, "Modified Crown Ether Catalysts. 3. Structural Parameters Affecting Phase Transfer Catalysis by Crown Ethers and a Comparison of the Effectiveness of Crown Ethers to that of Other Phase Transfer Catalysts," J. Am. Chem. Soc., 102, 4810(1980); S. T. Jolley and J. S. Bradshaw, "Synthesis of Diethyl, Di-n-Octyl, and Mono- and Dicyclohexano Macrocyclic Polyether-Diester Ligands," J. Org. Chem., 45, 3554(1980); R. Perraud, H. Handel, and J.-L. Pierre, "Effects of Cryptands and Activation of Bases. VI. Reactions of Organolithiums, "Bull. Soc. Chim. France, 283(1980).

The above synthetic methods are of the second type and require the use of special substrates having reactive moieties or bulky substituents which must be carried through multiple synthetic steps and which may lead to the unwanted presence of the special substrate in the final product. Further, these processes may require the use of special and often times expensive stoichiometric reagents which, although not incorporated into the product, may limit the various alternatives available in the choice of the final product, i.e. substituted macrocyclic.

The aforementioned synthetic methods are to be distinguished from a method which employs the macroyclic compounds as the substrate in a single step synthesis to produce substituted macrocyclic compounds. Such a synthetic method may be achieved by employing a radical producing reagent in a reaction mixture with a macrocyclic compound. The photochemical reactions of 18-crown-6 and acetophenone disclosed in "Photochemistry of Host-Guest Complex I, Photochemical Reaction of Alkyl aryl ketones with 18-Crown-6", Tada, M. and Hirano, H., Tetrahedron Letters, 51, 5111-5114 (1978), wherein photons are employed (450 W high pressure Hg lamp) is such a method for producing substituted macrocylics which probably involves the use of non-catalytic amounts of photons to carry out the synthesis.

The use of the chemical initiator di-tert butyl peroxide in the reaction of 12-Crown-4, 15-Crown-5 and 18-Crown-6 with α-(tert-butyl-thio)-acrylonitrile is disclosed by S. Mignani and H. G. Viehe, unpublished results reported in H. G. Viehé, R. Merényi, L. Stella, and Z. Janousek, "Captodative Substituent Effects in Syntheses with Radicals and Radicophiles", Viehe, H. G., et al., Angew. Chem. Int. Ed. Engl. 18, 917-932, (Dec. 21, 1979), wherein the reaction of radicals with radicophilic olefins is diclosed.

According to the invention, it has now been found that substituted macrocyclic compounds can be prepared directly from multidentate macrocyclic compounds by free radical addition of substituent-forming compounds (saturated or unsaturated) in the presence of a chemical initiator. Suitable macrocyclics include all those disclosed in the above references having a carbon-hydrogen bond of a ring carbon adjacent to a hetero atom,/* i.e. 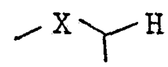 where X is the hetero atom. Typical macrocyclics include crown ethers, cryptands, and the like. Typical unsaturated compounds include olefins, carbynes, nitrenes, silylenes and the like. Typical saturated compounds include halogen, halogen containing compounds, another crown ether or other ether, and the like, which are capable of forming radicals in the reaction system in the presence of a chemical initiator. Suitable chemical initiators include those initiators which will abstract a hydrogen atom from the macrocyclic. Typical chemical initiators useful in the process are peroxides, peracids, peresters, peranhydrides, persulfates, various azo compounds and various triplet state molecules.

The multidentate macrocyclics which may now be considered as substrates for reactions with substituent-forming compounds (saturated or un-

/* (preferably oxygen, nitrogen or sulfur, specially oxygen)

saturated) include high molecular weight cyclics such as Crown ethers, high molecular weight aza macrobicyclic polyethers (cryptands), polyethers containing sulfur (thia Crown compounds), and the like, wherein exists at least one carbon-hydrogen bond of a ring carbon adjacent to a hetero atom as a reaction site for further chemical elaboration.

In contrast to the use of these macrocyclics as catalysts or inert complexing agents, it has been found that these macrocylics are particularly useful as substrates for reaction with saturated and unsaturated compounds in the presence of a chemical initiator. The substituted macrocyclics produced thereby may be employed in many areas including surfactant mixtures, catalysts, solvents and the like.

With respect to the selection of macrocyclic compounds embodied within the above descriptions, the most preferred are those characterized as Crown ethers, most preferably those containing at least four (4) hetero oxygen atoms in the ring portion.

For an ample description of Crown ethers, their structures and nomenclature, reference is made to the aforementioned text, "Synthetic Multidentate Macrocyclic Compounds", edited by Reed M. Izatt and James J. Christensen. The Crown ethers described therein may be employed in this invention to the extent that they meet the structural limitations

recited herein for the macrocyclic compounds of this invention.

A secondary preferred class of substrates is cyclic compounds termed cryptands (aza macrobicyclic polyethers), as described in the aforementioned text, "Synthetic Multidentate Macrocylic Compounds", edited by Reed M. Izatt and James J. Christensen and to the extent those described meet the structural limitations recited herein for the macrocylic employed in this invention.

The substituent-forming compound employed in the invention can be either an unsaturated or saturated compound. The use of the term "unsaturated" herein refers to compounds which are coordinatively unsaturated in that they have unfilled valence positions whereas "saturated" refers to compounds that have filled valence positions. The unsaturated or saturated compounds employed herein are "Substituent-forming compounds" as that term is employed herein and are characterized by their ability to form radicals in the reaction system in the presence of a chemical initiator.

The term "alkyl" as used herein refers to straight chain and branched alkyl groups, including substituted alkyl groups, having at least 2 carbon atoms, the preferred alkyl groups having from about 8 to about 30 carbon atoms with the most preferred containing from about 8 to about 20 carbon atoms.

Unsaturated compounds which may be added to the aforementioned macrocyclics according to the

invention include, but are not limited to, olefins, substituted olefins, allenes, conjugated dienes, unconjugated dienes, acetylenes, carbon monoxide, carbonyl containing compounds, carbenes, carbynes, nitrenes, silylenes and the like. The preferred unsaturated compounds are $\alpha$-olefins having from 8 to 30 carbon atoms and most preferably from 8 to 20 carbon atoms, e.g. 1-decene. The aforementioned unsaturated compounds may also contain one or more moieties, e.g., halide, sulfate and the like, other than the moiety giving rise to the unsaturation or ability to form a radical in the reaction system.

Saturated compounds which may be added to the aforementioned macrocyclics include those compounds capable of forming radicals in the reaction system and include halogens, halogen acids, halogen containing compounds (e.g., $CCl_4$, $CHCl_3$, $CH_3CCl_3$, N-bromosuccinimide), Crown ethers, ethers, primary and secondary alcohols, certain metal complexes (e.g., $Mn_2(CO)_{10}$), and the like. The ability to form radicals under reaction conditions and in the presence of a chemical initiator is common to the above-noted substituent-forming compounds and any compound that may form a radical under the reaction conditions may be employed.

Chemical initiators which may be used in the invention are those initiators which will abstract a hydrogen from the carbon-hydrogen bond of a ring carbon adjacent to a hetero atom including, but not limited to, peroxides, peracids, peresters, peranhydrides,

certain azo compounds and various triplet state molecules, e.g., $O_2$. Preferred chemical initiators include organic peroxides such as lauroyl peroxide, acetyl peroxide, peracetic acid, t-butyl hydroperoxide, cumene hydroperoxide, azo (bis-isobutyro) nitrile,benzoyl peroxide, tert-butyl peroxyacetate, methyl ethyl ketone peroxide, di-tert-butyl peroxide, and the like as well as other commercially available chemical initiators. In addition, many of the inorganic persalts can be used, e.g. perborates, percarbonates and persulfates.

The relative ratio of the macrocyclic to substituent-forming compound in the reaction-mixture may range from about 1:300 to about 300:1 preferably from about 1:100 to about 100:1 and most preferably from about 1:20 to about 20:1.

The amount of chemical initiator employed in the reaction mixture depends in part on the substituent-forming compound selected. The concentrations of the chemical initiator will generally be present in an amount from about 0.001 to about 100 mole percent, based on moles of the substituent-forming compound, preferably from about 0.1 to about 30 mole percent and most preferably from about 0.1 to 10 mole percent. Depending on the substituent-forming compound selected a catalytic or non-catalytic amount of chemical initiator will be employed.

The process of this invention may be carried out without a solvent since the macrocyclic compounds employed herein will function as the solvent. If

desired, a solvent may be employed to carry out the process. Typical solvents deemed suitable include t-butanol, benzene, water and the like.

The time required for carrying out the process is a function of the selected macrocyclic, substituent-forming compound and selected reaction conditions. The time may vary from a few seconds to many hours depending in part on the decomposition rate of the chemical initiator. For a continuous process a reaction time of from a few seconds to about an hour is preferred.

The process should be carried out under conditions chosen to prevent the chemical initiator from decomposing too rapidly and thus limiting unwanted side reactions. The temperature is generally selected to be about 0°C to about 400°C with from about 50°C to about 300°C being preferred and from about 50°C to about 200°C being most preferred.

The process of this invention is advantageously conducted in a closed system wherein loss of the macrocyclic and/or substituent-forming compound by evaporation is minimized or avoided in addition to which air and moisture are excluded. As a convenience, the pressure at which the reaction is conducted may be the pressure created by the system, i.e., autogeneous pressure. If desired, however, higher or lower pressure may be employed depending in part on the substituent-forming compound selected.

It is to be emphasized that in any reaction system the ratio of macrocyclic to substituent forming compound, temperature and pressure selected, the selection and concentration of chemical initiator each play a role in determining the conditions under which the process is carried out.

Various methods of practicing the invention are illustrated by the following examples. These examples are intended to illustrate that the invention can be practiced.

EXAMPLE 1

The reaction of macrocyclic and radical forming compounds was demonstrated by placing 0.54 mole of 15-Crown-5 and 0.11 mole of 1-decene into a 200 ml. round-bottomed flask and introducing nitrogen therein. The flask was attached to a distillation head, immersed in an oil bath and heated with stirring (magnetic stirrer) to about 146°C. A chemical initiator, di-t-butyl peroxide, in the amount of 0.01 mole, was added to the reaction mixture by syringe through a septum. A slight exotherm was observed after which the reaction mixture was maintained at about 146°C for about 4.5 hours.

The flask containing the reaction mixture was cooled and the contents were vacuum distilled. Two fractions were collected at 95°C/0.20 mm and 98°C/0.15 mm Hg and the combined fractions contained 95.86 grams. A third fraction was collected (using a diffusion pump) containing 2.42 grams to provide a total of 98.28 grams. The stoichiometric excess of 15-Crown-5-is readily accounted for and may be re-used without working to remove the initiator-derived t-butanol and acetone.

The distillation residues were tested by use of proton nuclear magnetic resonance (showing only carbon-bonded protons beta to ether oxygen and aliphatic carbon, no olefinic, aldehydic, hydroxyl, acid, or methoxy protons) and by infra red spectroscopy (showing no hydroxyl or carbonyl), and by chemical ionization mass spectroscopy showing a major peak at 361 (molecular weight of 15-crown-5 + 1-decene + 1). The above results are consistent with the presence of n-decyl-15-crown-5.

EXAMPLE 2

The procedure of Example 1 was repeated except that a 250 ml round-bottom flask was employed, the reaction mixture was heated to about 123°C, and after addition of the di-t-butyl peroxide, the reaction mixture was maintained at about 123°C for about 60 hours. Two fractions were obtained from the reaction mixture by vacuum distillation (up to 118°C/ 0.25 mm Hg), containing a total of 99.95 grams. The residue was transferred to another flask and distilled further by use of a diffusion pump. Two additional fractions containing 2.29 grams and 0.18 grams were obtained.

Analysis of the residue, as described in Example 1, gave results consistent with the presence of n-decyl-15-crown-5.

EXAMPLE 3

The reaction of 18-Crown-6 (0.50 mole) and 1-tetradecene (0.10 mole) was demonstrated by repeating the procedure of Example 1 except that the flask was heated to about 123°C. Di-*t*-butyl peroxide (0.01 mole) was added to the reaction mixture by syringe through a septum. A slight exotherm was observed, after which the reaction mixture was maintained at a temperature of about 123°C for about 43 hours.

The flask containing the reaction mixture was cooled and vacuum distilled by use of a diffusion pump. Two fractions were collected at 129°C/0.02 mm Hg and at 104°C/0.005 mmHg of 114.96 grams and 3.85 grams, respectively, containing predominantly 18-crown-6.

The distillation residue was analyzed and showed only carbon-bonded protons beta to ether oxygen and aliphatic carbon when analyzed by proton n.m.r. spectroscopy, only carbon alpha to aliphatic carbon and ether oxygen when analyzed by $^{13}C$ n.m.r. spectroscopy, and no hydroxyl or carbonyl absorptions when analyzed by infrared spectroscopy. Analysis by chemical ionization mass spectroscopy showed peaks at 461 and 657 consistent with the presence of both 1:1 and 1:2 adducts of 18-crown-6: olefin, i.e., consistent with the production of n-tetradecyl-18-crown-6 and 18-crown-6 with higher aliphatic content.

WHAT IS CLAIMED IS:

1) A process for the preparation of substituted macrocyclic compounds which comprises the reaction of a macrocyclic compound having a carbon-hydrogen bond adjacent to a hetero ring atom, and a substituent-forming compound having at least 2 carbon atoms in the presence of a chemical initiator.

2) The process of claim 1 wherein the substituent-forming compound is an α-olefin, preferably an olefin which contains about 8 to about 30, preferably 8 to 20, carbon atoms.

3) The process of claim 1 or 2 wherein the macrocyclic compound is a Crown ether or a cryptand.

4) The process of claim 1 or 3 wherein the substituent-forming compound is a saturated or an unsaturated compound.

5) The process according to any one of the preceding claims wherein the mole ratio of macrocyclic compound to substituent-forming compound is from about 1:100 to about 100:1, preferably from about 1:20 to about 20:1.

6) The process according to any one of the preceding claims wherein the chemical initiator is an organic peroxide, preferably di-t-butyl peroxide.

7) The process according to any one of the preceding claims wherein the Crown ether is 15-Crown-5 or 18-Crown-6.

8) The process according to any one of the preceding claims wherein a catalytic amount of chemical initiator is employed, preferably in an amount from about 0.01 to about 30 mole percent.

9) The process according to any one of the preceding claims

wherein the process is carried out at a temperature of from about 0°C to about 400°C.

10) The process for the preparation of an alkyl substituted Crown ether, preferably 15-Crown-5, by the reaction of said Crown ether and an α-olefin having at least 3 carbon atoms, characterized that the process is carried out in the presence of a catalytic amount of chemical initiator.

11) Process according to claim 11 where said α-olefin contains from about 10 to about 30 carbon atoms.

0054283

European Patent Office

# EUROPEAN SEARCH REPORT

Application number
EP 81110377.9

## DOCUMENTS CONSIDERED TO BE RELEVANT

| Category | Citation of document with indication, where appropriate, of relevant passages | Relevant to claim |
|---|---|---|
| A | US - A - 3 987 061 (PEDERSEN)<br>* Abstract *<br>-- | 1 |
| A | US - A - 3 833 491 (KENNEDY)<br>* Claim 2 *<br>-- | 1 |
| A | EP - A1 - 0 004 863 (L'OREAL)<br>* Abstract *<br>& US-A-4 297 102 (27-10-1981)<br>-- | 1 |
| A | CHEMICAL ABSTRACTS, vol. 91, no. 4, July 23, 1979, Columbus, Ohio, USA<br>M. TAKAGI "Chemistry of poly-(macrocycle)-chemistry of crown ethers and cryptands"<br>page 776, column 1, abstract no. 31971a<br>& Kaga Ku no Ryoiki, vol. 33(2), 1979, pages 119-131<br>---- | 1 |

CLASSIFICATION OF THE APPLICATION (Int. Cl. 3)

C 07 D 323/00
C 07 D 498/08
C 07 D 273/00
C 07 D 327/00

TECHNICAL FIELDS SEARCHED (Int.Cl. 3)

C 07 D 323/00
C 07 D 273/00
C 07 D 327/00
C 07 D 498/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| VIENNA | 26-02-1982 | BRUS |